(19) Europäisches Patentamt
European Patent Office
Office européen des brevets

(11) Publication number: **0 192 767**
**B1**

# (12) EUROPEAN PATENT SPECIFICATION

(45) Date of publication of patent specification: **16.05.90**

(21) Application number: **85905012.2**

(22) Date of filing: **11.07.85**

(86) International application number:
**PCT/US85/01312**

(87) International publication number:
**WO 86/01511 13.03.86 Gazette 86/06**

(51) Int. Cl.[5]: **C 07 F 7/08,** C 07 C 63/72, C 07 C 47/55, C 07 C 22/00, C 07 D 303/08, C 07 C 25/18, C 08 G 59/38, C 08 K 5/00

## (54) DERIVATIVES OF DIPHENYLHEXAFLUOROPROPANE.

(30) Priority: **04.09.84 US 646999**

(43) Date of publication of application:
**03.09.86 Bulletin 86/36**

(45) Publication of the grant of the patent:
**16.05.90 Bulletin 90/20**

(84) Designated Contracting States:
**CH DE FR GB IT LI NL SE**

(56) References cited:
**EP-A-0 030 432**
**EP-A-0 092 310**
**GB-A-1 228 007**
**US-A-3 310 573**

**Chemical Abstracts, vol. 75, no. 3, 19 July 1971, Columbus, Ohio, (US), see page 419, abstract no. 19893p**

**Journal of Polymer Science-Polymer Chemistry Edition, vol. 20, 1982, New York, (US) K.S.Y. Lau et al.:"Synthesis of polymer-intermediates containing the hexafluoroisopropylidene group via the functionalization of 2,2-diphenylhexafluoropropane", pages 2381-2393, see page 2381**

(73) Proprietor: **Hughes Aircraft Company**
**7200 Hughes Terrace P.O.Box 45066**
**Los Angeles California 90045-0066 (US)**

(72) Inventor: **LAU, Kreisler, S., Y.**
**1708 South Cordova Street**
**Alhambra, CA 91801 (US)**
Inventor: **KELLEGHAN, William, J.**
**10432 Lundene Drive**
**Whittier, CA 90601 (US)**

(74) Representative: **Kuhnen, Wacker & Partner**
**Schneggstrasse 3-5 Postfach 1553**
**D-8050 Freising (DE)**

(56) References cited:
**Grant & Hackh's Chemical Dictionary (5th Ed.), pages 24,216 and 513; (4th Ed.), pages 24 and 247**

**Nomenclature of Organic Chemistry IUPAC (1979), pages 11-18**

**EP 0 192 767 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

## BACKGROUND OF THE INVENTION

1. Field of the Invention

The present invention relates generally to diphenylhexafluoropropane derivatives and, more particularly, to derivatives wherein various groups are attached to the para-position on the phenyl rings of the diphenylhexafluoropropane by way of a carbon or silicon attachment.

2.. Description of the Background Art

Diphenylhexafluoropropane is a very versatile compound which basically consists of two phenyl rings which are attached on opposite sides of the number two carbon of hexafluoropropane. In the past, a number of different derivatives of diphenylhexafluoropropane have been produced by attaching various different functional groups at either the para- or the meta- positions on the phenyl rings. These derivatives have proven to be useful intermediates in the synthesis of thermally stable resins for use in high temperature structural composites.

Diphenylhexafluoropropane compounds have been produced where various different functional groups, such as ethynyl groups, amino groups, or halogens, are attached to the phenyl rings at the meta-position by way of carbon or nitrogen linkages. As is well known, the specific isomeric form of chemical precursors and intermediates is an important factor in determining the final characteristics and performance of the desired chemical product. Accordingly, it has been desirable to also prepare diphenylhexafluoropropane derivatives in which the functional groups are attached to the phenyl rings at the para- position instead of the meta- position.

Diphenylhexafluoropropane compounds having an ethynyl group attached at the para- or meta-position have been developed and are disclosed in U.S. Patent No. 4,374,291, which is assigned to the present assignee. However, other para-substituted diphenylhexafluoropropane derivatives which have been developed in the past and which are presently available all include an oxygen or sulfur linkage between the functional group and the phenyl ring. The attachment of the functional groups to the para-position on the phenyl rings via sulfur or oxygen results in a relatively flexible linkage which, in turn, tends to produce relatively flexible polymer products. Although flexible high temperature polymers are well suited for a variety of applications, it is many times desirable to provide thermally stable resins which have increased rigidity. Further, sulfur and oxygen linkages in polymer resins are more susceptible to oxidative degradation or breakdown resulting in premature deterioration of the polymer in which the sulfur or oxygen linked diphenylhexafluoropropane derivative is incorporated.

Therefore, there is a present need to provide para-substituted diphenylhexafluoropropane derivatives which can be used as alternate polymer precursors to meta-substituted derivatives and which do not include a sulfur or oxygen linkage between the para-substituted group and the phenyl rings.

## SUMMARY OF THE INVENTION

In accordance with the present invention, a new group of para-substituted diphenylhexafluoropropane compounds are disclosed which provide an alternative polymer precursor to the meta-substituted diphenylhexafluoropropane derivatives while not requiring an oxygen or sulfur linkage.

The new diphenylhexafluoropropane derivatives have the following formula:

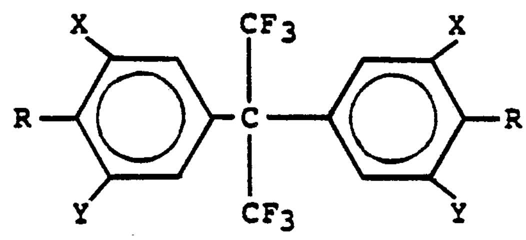

where R is an alkyl, alkylene, carbonyl, carboxy, acyl, aryl, epoxy, silyl or siloxy group; and where X and Y are hydrogen or halogen.

The diphenylhexafluoropropane derivatives in accordance with the present invention have a direct carbon or silicon linkage between the functional groups R and the phenyl rings of the diphenylhexafluoropropane. Carbon or silicon linkages are more rigid than sulfur or oxygen. As a result, more rigid polymers can now be produced from para-substituted diphenylhexafluoropropane than was previously possible with prior sulfur and oxygen linked derivatives. The new diphenylhexafluropropane derivatives are not only useful in preparing rigid polymers, but the resulting polymers are also thermally stable and resistant to oxidative attack due to the absence of sulfur or oxygen linkages.

The above-discussed and many other features and attendant advantages of the present invention will become better understood by reference to the following detailed description of the invention.

The compounds in accordance with the present invention have the following general formula

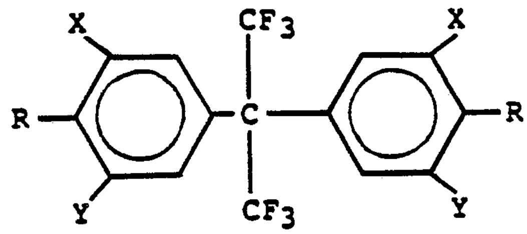

where R is: (a) an unsubstituted or substituted alkenyl; (b) an epoxyalkyl, or (c) a trialkylsilyl group; and where X and Y are hydrogen or a halogen.

The documents Chem. Abs. 75:19893, US—A—3,310,573, GB—A—1,228,007, EP—A—30 432, EP—A—92 310 and J. Polymer Sci. Vol. 20, (1982) pages 2381 to 2393 in part are related to compounds of similar type as the present invention. However, they neither disclose nor suggest the derivatives of diphenylhexafluoropropane in which the vinyl rings are substituted with an alkenyl, and epoxyalkyl or a trialkylsilyl group.

Preferred alkenyl groups include from 2 to 4 carbon atoms. Vinyl and vinyl derivative groups are particularly preferred. These groups include vinyl, vinyl chloride, propylene, vinyl acetate, styrene, isobutylene, vinylidene chloride or methylmethacrylate and modified acrylics.

Epoxyalkyl having from 1 to 4 carbon atoms and trimethylsilyl being preferred. In addition, the abovementioned alkenyl group may include one or two phenyl rings.

In addition to the R groups attached at the para-position, the new compounds may include a halogen substituted at the meta-position. Any of the halogens may be used with iodine and bromine being preferred.

Both X and Y may be a halogen if desired; however, it is preferred that when X is a halogen, Y is hydrogen. Also preferred are compound where both X and Y are hydrogen.

The preparation of compounds in accordance with the present invention preferably begins with 2,2-bis(4-bromophenyl)hexafluoropropane (BPHP). BPHP is a reactive monomer which is the subject of U.S. Patent No. 4,503,254, which has been assigned to the same assignee of the present invention. Although the preferred starting material is bromine substituted, other para-halogenated substituted compounds, such as 2,2-bis(4-iodophenyl)hexafluoropropane and 2,2-bis(4-chlorophenyl)hexafluoropropane, may be used.

The 2,2-bis(4-bromophenyl)hexafluropropane which is the starting compound in most of the following examples is prepared by reacting triphenylphosphine dibromide with Bisphenol AF [2,2-bis(4-hydroxphenyl)hexafluropropane] at temperatures above 280°C. Bisphenol AF is a common compound which is widely available commercially. An exemplary synthesis is as follows:

To a slurry of triphenylphosphine dibromide (0.2 mole) in dichloromethane (250 ml) under argon was added 2,2-bis(4-hydroxyphenyl)hexafluoropropane (0.1 mole). The solvent was removed by distillation to leave a solid reaction mixture, and the flask which contained the reaction mixture was placed in a molten metal bath at 350°C for two hours. The reaction mixture was cooled to 100°C and poured into another flask. Further cooling of the reaction mixture resulted in a solid within the flask which was washed three times with 300 ml portions of hexane, and the hexane solution was filtered to remove unwanted reaction byproducts. The resulting hexane solution of the product was washed with 20% sodium hydroxide and deionized water. The solution was then dried over anhydrous magnesium sulfate and passed down a short alumina column. The hexane was removed from the solution and the resulting semi-solid was distilled to produce a 76% yield of the product 2,2-bis(4-bromophenyl)hexafluoropropane, which is a high yield for reactions of this nature.

Examples of preparation of preferred diphenylhexafluoropropane derivatives are as follows:

Example 1

2,2-bis(4-trimethylsilylphenyl)hexafluoropropane

The synthesis is shown schematically as follows:

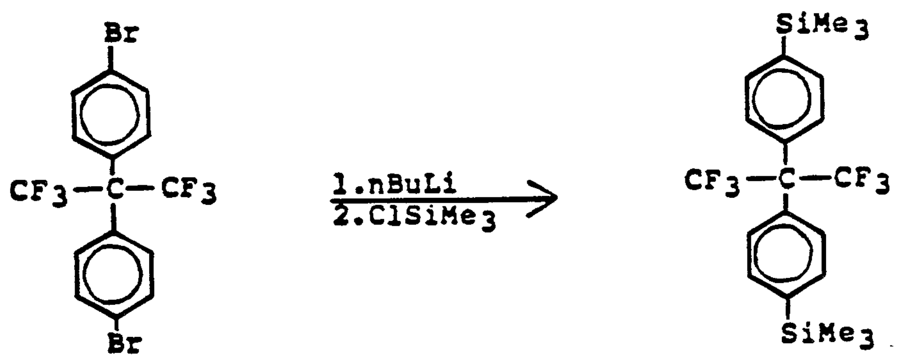

To a solution of 4.62 g (0.1 mole) 2,2-bis(4-bromophenyl)hexafluoropropane in 75 ml of anhydrous tetrahydrofuran was added dropwise 28 ml (0.4 moles) of a 1.4 molar solution of n-butyllithium in hexane while the reaction mixture was cooled in a dry ice-acetone slush. The reaction mixture was stirred for one half hour at −78°C. A dropwise addition of chlorotrimethylsilane (5 ml, 0.04 moles) was then made to the reaction mixture at −78°C. The stirring slurry was allowed to come to ambient temperature over 16 hours. To the reaction mixture was added 100 ml hexane. The organic layer was washed with 2 × 150 ml saturated sodium chloride solution and 2 × 150 ml water. The organic layer was dried, filtered, and concentrated to yield the white crystalline product 2,2-bis(4-trimethylsilylphenyl)hexafluoropropane.

The following physical data were recorded:

NMR ($CDCl_3$): δ 7.4 (m, 4H, aromatic) and 0.5 ppm (s, 9H, silylmethyl).

IR (film): 2970, 1325, 1255, 1210, 1180, 970, 840, 815 $cm^{-1}$.

Example 2

2,2-bis(4-vinylphenyl)hexafluoropropane

The synthesis is shown schematically as follows:

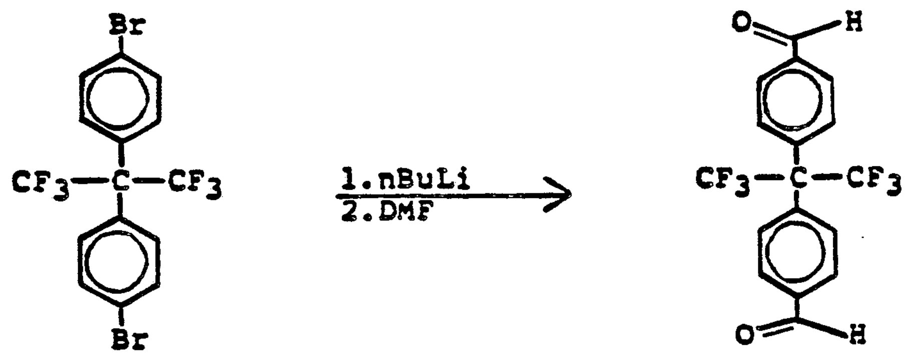


(I)

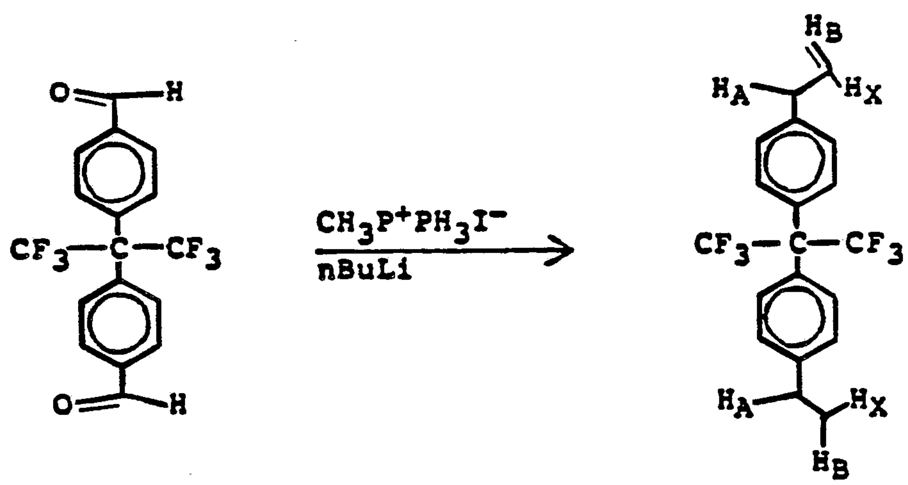


(II)

To a solution of 17.58 g (0.38 moles) of 2,2-bis(4-bromophenyl)hexafluoropropane in 100 ml anhydrous tertrahydrofuran (THF) at −70°C was added dropwise 83 ml of a 1.1 molar solution in hexane of n-butyllithium. The addition took thirty minutes. After 15 minutes at −60 to −65°C, a dropwise addition of 10.0 g (0.137 mole) of dimethylformamide in 50 ml anhydrous tetrahydrofuran was made to the reaction mixture. The reaction mixture was warmed to 25°C over one hour and maintained at 25°C for eighteen hours. At the end of this period, the reaction mixture was poured into 1 liter of stirred water. The product was extracted with ethyl ether, washed with 2 × 200 ml water and the ethereal layer dried. Concentration of the ethereal fraction afforded 8.46 g (23.5 moles, 61.8% yield) of the white solid product 2,2-bis(4-formylphenyl)hexafluoropropane. M.P. 130—132°C.

The following physical data were recorded:

NMR ($CDCl_3$): δ 10.1 (s, 1H, aldehydic proton), and 7.7 ppm (m, 4H, aromatic).

IR (KBr): 1700, 1385, 1315, 1280, 1250, 1230, 1210, 1190, 1170, 1115, 810 $cm^{-1}$.

| Elemental Analysis: | | C | H | F | Br |
|---|---|---|---|---|---|
| $C_{17}H_{10}F_6O_2$ (360.253) | Calc. | 56.68 | 2.80 | 31.64 | 0.0 |
| | Found | 56.82 | 2.90 | 31.47 | 0.23 |

Methyl triphenylphosphonium iodide (8.08 g) was suspended in 50 ml anhydrous tetrahydrofuran under argon. After cooling to −78°C the slurry was treated with 20 ml (24 mMoles) of 1.2M n-butyllithium. After stirring for 15 minutes, a solution of the dialdehyde product disclosed above in 40 ml tetrahydrofuran was added to the slurry still at −78°C. After completion of the addition, the reaction mixture was warmed to 25°C over two hours and maintained at this temperature for 20 hours. The reaction mixture was poured into 500 ml of 10% hydrochloric acid. The product was extracted by four 100 ml ether washes. The combined ethereal extracts were washed with 200 ml of saturated sodium chloride solution and dried over magnesium sulfate. Concentration of the dried ether solution yielded a yellow oil. Column chromatography of this oil using silica gel and 10% methylene chloride in hexane yielded 1.370 g (38.5% yield) of a clear yellow viscous oil which was determined to be 2,2-bis(4-vinylphenyl)hexafluoropropane.

The following physical data were recorded: GC—MS: one peak, m/e 356

NMR ($CDCl_3$): δ 7.3 (bs, 4H, aromatic H's), 6.9, 6.7, 6.6, 6.5 (d × d, 1H, $J_{AB}$=17.5Hz, $J_{AC}$=11Hz, vinyl $H_A$), 5.8, 5.6, (d × d, 1H, $J_{AB}$=17.5Hz, $J_{BC}$=1.5Hz, vinyl $H_B$), and 5.3, 5.1 ppm (d × d, 1H, $J_{AC}$=11Hz, $J_{BC}$=1.5Hz, vinyl $H_C$). The three vinyl protons give a classical ABX three-nuclei splitting pattern.

Example 3

2,2-bis(4-epoxyethylphenyl)hexafluoropropane

The synthesis is shown schematically as follows:

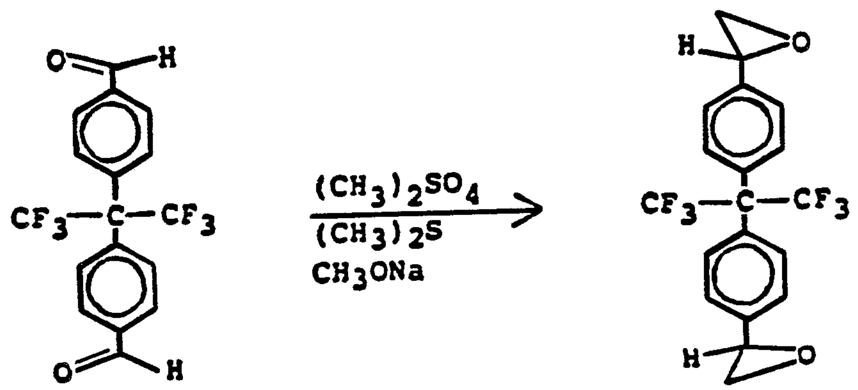


In a flask with an argon atmosphere was placed 3.78 g (0.03 mole) dimethyl sulfate, 1.05 g (0.033 mole) dimethyl sulfide, and 20 ml acetonitrile. The mixture was stirred 24 hours at 25°C. Sodium methoxide (1.87 g, 0.033 mole) was added in one portion and the reaction mixture stirred another hour at 25°C. To the reaction mixture was then added 3.60 g (0.01 mole) 2,2-bis(4-formylphenyl)hexafluoropropane, formed as in Example 2, over 15 minutes. After the addition was complete, the stirring was maintained for 18 hours. The reaction flask was then equipped for distillation and the majority of the dimethyl sulfide and dimethyl sulfate removed. To the cooled reaction product was added 20 ml acetonitrile. The organic layer was washed with 4 × 100 ml deionized water, dried over magnesium sulfate, concentrated, and purified using a Kugelrohr apparatus to obtain 2.21 g, 0.0057 moles, 57% yield of the product 2,2-bis(4-epoxyethylphenyl)-hexafluoropropane.

The following physical data were recorded:

IR (film): 1270, 1250, 1215, 1185, 975, 885, 835 $cm^{-1}$.

NMR ($CDCl_3$): δ 7.3 (s), 5.6 (s), 3.8 (m), 3.1 (m), 2.7 (m) ppm.

Example 4

2,2-bis(3-iodo-4-methylphenyl)hexafluoropropane

The synthesis is shown schematically as follows:

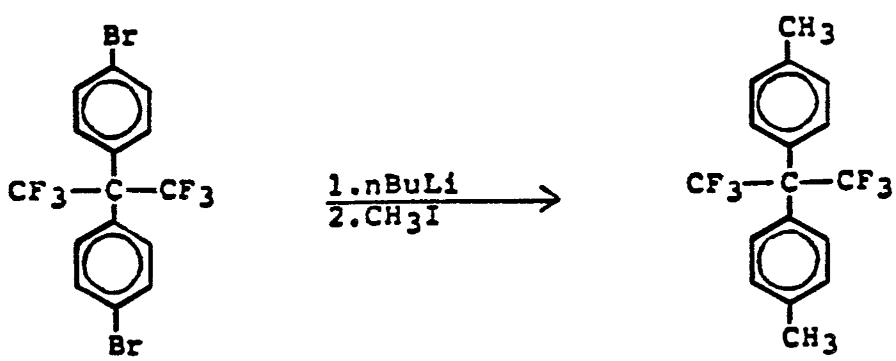

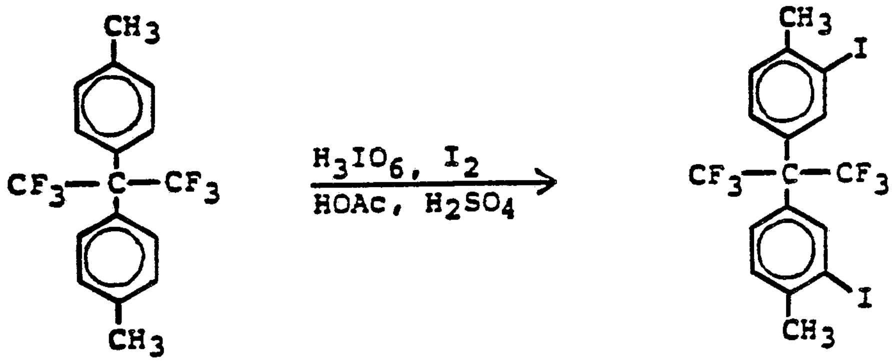


A solution was prepared containing 58.78 g (0.127 mole) 2,2-bis(4-bromophenyl)hexafluoropropane in 250 ml anhydrous tetrahydrofuran under an argon atmosphere. The solution was cooled to −78°C in a dry ice-acetone slush bath. To the cooled solution was added dropwise 0.28 mole n-butyllithium (112 ml of a 2.5 molar solution in hexane) over a 15 minute period. The solution was stirred for 30 minutes at approximately −78°C after the addition was complete. Cooling of the reaction mixture was continued while adding dropwise 39.7 g (0.28 moles) methyl iodide over a 30 minute period. Stirring and cooling was maintained for one hour after the completion of the addition. Then the slush bath was removed and the reaction mixture gradually warmed to ambient temperature. The reaction mixture was washed with 2 × 250 ml of each of the following: saturated sodium chloride solution, dilute sodium bisulfite solution, and deionized water. The organic layer was concentrated to an oil. Recrystallizing the oil from hexane cooled to −78°C yielded 28.25 g (0.085 moles, 67% of theory) of the product 2,2-bis(4-methylphenyl)hexafluoropropane. M.P. 76—77°C.

The following physical data were recorded:
IR (KBr): 2990, 2970, 1525, 1265, 1215, 1180 $cm^{-1}$.
NMR ($CDCl_3$): δ 7.2 (s), 2.3. (s) ppm.

| Elemental Analysis: | | C | H | F |
|---|---|---|---|---|
| $C_{17}H_{14}F_6$ (332.287) | Calc. | 61.45 | 4.25 | 34.30 |
| | Found | 6.64 | 4.40 | 34.51 |

In a reaction flask equipped with a condenser, 2,2-bis(4-methylphenyl)hexafluoropropane (9.960 g), prepared as above, iodine (12.304 g), periodic acid (5.785 g), 150 ml glacial acetic acid, 10 ml water, and 3 ml of sulfuric acid were stirred vigorously and heated at 85—90°C for 72 hours. The reaction mixture was cooled and poured into 500 ml of water. The aqueous reaction mixture was extracted with 300 ml of ether. The ethereal phase was washed with sodium bisulfite solution to remove iodine, then 200 ml of water, 200 ml saturated bicarboante solution, 200 ml of water, and finally dried over anhydrous magnesium sulfate. Concentration of the ethereal phases and recrystallization from pentane yielded the product 2,2-bis(3-ido-4-methylphenyl)-hexafluoropropane, a white powdery solid. M.P. 101—102°C, in 45% yield.

The following physical data were recorded:
NMR ($CDCl_3$): δ, 7.8 (s), 7.2 (s), 2.4 (s) ppm.
MS: (70 eV) m/e 584.

| Elemental Analysis: | | C | H | F |
|---|---|---|---|---|
| $C_{17}H_{12}F_6I_2$ (584) | Calc. | 34.96 | 2.07 | 43.45 |
| | Found | 35.27 | 2.08 | 43.23 |

The diphenylhexafluoropropane derivatives of this invention are useful in a large number of applications. The alkenyl and epoxyalkyl derivatives may be used alone to produce resin systems or they may be blended with known commercial resins such as any of the vinyl, styrene, butadiene or similar common resins. The ease of vinyl and epoxy polymerization and the special structural characteristics of the vinyl (Example 2) and epoxy- (Example 3) derivatives of the present invention jointly provide a facile entry to vinyl and epoxy resins which have the desirable low dielectric constant and low dissipation loss properties for electronic applications.

In addition, the derivatives may be useful as reactive intermediates in the production of fluorocarbon based pharmaceuticals as well as other organic products.

Exemplary polymers which can be made utilizing the diphenylhexafluoroprpane derivatives and the methods for preparing them are as follows:

Example 5

Preparation of polybenzisoxazole containing the hexafluoropropane group

A low molecular weight polymer was prepared by reacting 2,2-bis(4-formylphenyl)hexafluoropropane (see Example 2) and 2,2-bis-(3-amino-4-hydroxyphenyl)hexafluoropropane in polyphosphoric acid at 200°C for 4 hours. The latter compound was prepared as described in the publication by K.S.Y. Lau, et al., entitled "Synthesis of Polymer Intermediates Containing the Hexafluoroisopropylidene Group via Functionalization of 2,2-diphenylhexafluoropropane," in the *Journal of Polymer Science: Polymer Chemistry Edition,* Vol. 20, 1982, pages 2381—2393.

The mixture was poured into 1 liter of water to precipitate the polymer. After drying at 100°C for 24 hours, the polymer could be purified further by dissolving in N-isopropylpyrrolidinone (NIP) (available from GAF Corporation) and reprecipitating from a hexane-dichloromethane mixture. Inherent viscosity (measured in NIP) was 0.17.

Example 6

Copolymer of Epoxidized Polybutadiene and 2,2-bis(4-epoxyethylphenyl)hexafluoropropane

Commercially available epoxidized polybutadiene (from Nisso Company, 8% epoxidation of the 90% vinyl pendent groups) was blended with 20% of 2,2-bis(4-epoxyphenyl)hexafluoropropane prepared as in Example 3 and then cured inside a mold with 4% 1,2,3,6-tetrahydrophthalic anhydride and 1% dicumyl peroxide according to a standard multi-stage curing process to form a cured disc copolymer material.

**Claims**

1. A diphenylhexafluoropropane compound having the formula:

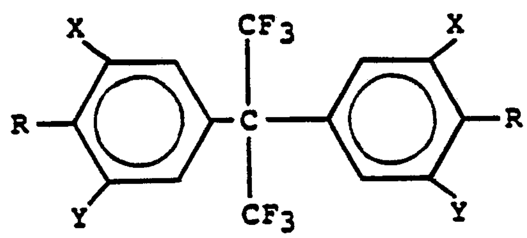

where R is: (a) an unsubtituted or substituted alkenyl; (b) an epoxyalkyl, or (c) a trialkylsilyl group; and where X and Y are hydrogen or a halogen.

2. A diphenylhexafluoropropane compound according to claim 1 where X is a halogen and Y is hydrogen.

3. A diphenylhexafluoropropane compound according to claim 1 where R is vinyl, β-chlorovinyl, β-methylvinyl, (β-acetoxy)vinyl, β-phenylvinyl, β,β-dimethylvinyl, α-chlorovinyl, or (β-methoxycarbonyl-β-methyl)vinyl.

4. A diphenylhexafluoropropane compound according to claim 1 where R is an alkenyl group having from 2 to 4 carbon atoms.

5. A diphenylhexafluoropropane compound according to claim 1 where R is trimethylsilyl.

6. A diphenylhexafluoropropane compound according to claim 1 where R is an epoxyalkyl group having from 2 to 4 carbon atoms.

7. A resin material comprising a mixture of a compound according to claim 3 or 6 and a polymer selected from the group consisting of polyethylene, polystyrene and polybutadiene.

8. A resin material comprising a mixture of 2,2-bis-(4-epoxyethylphenyl)hexafluoropropane according to claim 1 and epoxidized polybutadiene.

9. A polymer formed by polymerization of a compound according to claim 1.

**Patentansprüche**

1. Eine Diphenylhexafluorpropan-Verbindung mit der Formel:

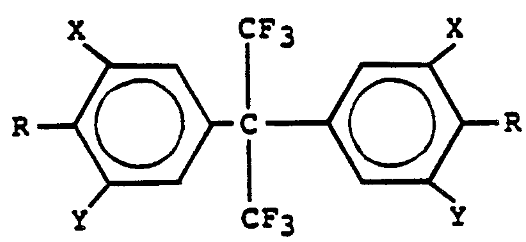

worin R: (a) ein unsubstituiertes oder substituiertes Alkenyl; (b) ein Epoxyalkyl oder (c) eine Trialkylsilylgruppe ist; und worin X und Y Wasserstoff oder ein Halogen sind.

2. Eine Diphenylhexfluorpropan-Verbindung nach Anspruch 1, worin X ein Halogen und Y Wasserstoff ist.

3. Eine Diphenylhexafluorpropan-Verbindung nach Anspruch 1, worin R Vinyl, β-Chlorvinyl, β-Methylvinyl, (β-Acetoxy)vinyl, β-Phenylvinyl, β,β-Dimethylvinyl, α-Chlorvinyl oder (β-Methoxycarbonyl-β-methyl)vinyl ist.

4. Eine Diphenylhexafluorpropan-Verbindung nach Anspruch 1, worin R eine Alkenylgruppe mit von 2 bis 4 Kohlenstoffatomen ist.

5. Eine Diphenylhexafluorpropan-Verbindung nach Anspruch 1, worin R Trimethylsilyl ist.

6. Eine Diphenylhexafluorpropan-Verbindung nach Anspruch 1, worin R eine Epoxyalkylgruppe mit von 2 bis 4 Kohlenstoffatomen ist.

7. Ein Harzmaterial, eine Mischung aus einer Verbindung nach Anspruch 3 oder 6 und einem Polymer umfassend, welches ausgewählt ist aus der Gruppe bestehend aus Polyethylen, Polystyrol und Polybutadien.

8. Ein Harzmaterial, eine Mischung aus 2,2-bis-(4-Epoxyethylphenyl)hexafluorpropan, nach Anspruch 1 und epoxidiertes Polybutadien umfassend.

9. Ein Polymer, erhalten durch Polymerisation einer Verbindung nach Anspruch 1.

## Revendications

1. Composé de diphénylhexafluoropropane, répondant à la formule:

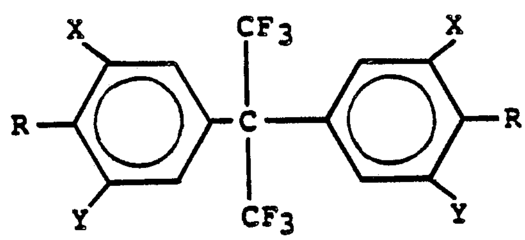

dans laquelle R représente: (a) un groupe alcényle non substitué ou substitué; (b) un groupe époxyalkyle ou (c) un groupe trialkylsilyle; et X et Y représentent de l'hydrogène ou un halogène.

2. Composé de diphénylhexafluoropropane suivant la revendication 1, dans lequel X est un halogène et Y est l'hydrogène.

3. Composé de diphénylhexafluoropropane suivant la revendication 1, dans lequel R est un groupe vinyle, β-chlorovinyle, β-méthylvinyle, (β-acétoxy)vinyle, β-phénylvinyle, β,β--diméthylvinyle, α-chlorovinyle ou (β-méthoxycarbonyl-β-méthyl)vinyle.

4. Composé de diphénylhexafluoropropane suivant la revendication 1, dans lequel R est un groupe alcényle ayant 2 à 4 atomes de carbone.

5. Composé de diphénylhexafluoropropane suivant la revendication 1, dans lequel R est le groupe triméthylsilyle.

6. Composé de diphénylhexafluoropropane suivant la revendication 1, dans lequel R est un groupe époxyalkyle ayant 2 à 4 atomes de carbone.

7. Résine comprenant un mélange d'un composé suivant la revendication 3 ou 6 et d'un polymère choisi dans le groupe comprenant un polyéthylène, un polystyrène et un polybutadiène.

8. Résine comprenant un mélange de 2,2-bis-(4-époxyéthylphényl)hexafluoropropane suivant la revendication 1 et un polybutadiène époxydé.

9. Polymère formé par polymérisation d'un composé suivant la revendication 1.